(19) Europäisches Patentamt
European Patent Office
Office européen des brevets

(11) Veröffentlichungsnummer: **0 330 016 A2**

# (12) EUROPÄISCHE PATENTANMELDUNG

(21) Anmeldenummer: **89102203.0**

(22) Anmeldetag: **09.02.89**

(51) Int. Cl.4: **C07D 405/06 , A01N 43/653 , A01N 43/50**

(30) Priorität: **20.02.88 DE 3805376**

(43) Veröffentlichungstag der Anmeldung: **30.08.89 Patentblatt 89/35**

(84) Benannte Vertragsstaaten: **AT BE CH DE ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Seele, Rainer, Dr.**
**Leiblstrasse 3**
**D-6701 Fussgoenheim(DE)**
Erfinder: **Himmele, Walter, Dr.**
**Eichenweg 14**
**D-6909 Walldorf(DE)**
Erfinder: **Karbach, Stefan, Dr.**
**Grundwiesenweg 44**
**D-6730 Neustadt(DE)**
Erfinder: **Sauter, Hubert, Dr.**
**Neckarpromenade 20**
**D-6800 Mannheim 1(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**

## (54) Neue Azolylmethyloxirane und diese enthaltende Fungizide.

(57) Azolylmethyloxirane der allgemeinen Formel I

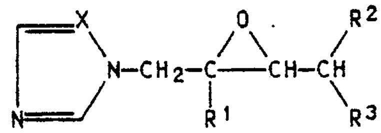

I ,

in welcher $R^1$, $R^2$ und $R^3$ gegebenenfalls Alkyl, Benzyl, Naphthyl, Biphenyl, Dioxanyl, Phenyl, Tetrahydropyranyl, Tetrahydrofuranyl, Norbornyl, $C_3$-$C_{12}$-Cycloalkyl oder $C_3$-$C_{12}$-Cycloalkenyl bedeuten,
X, CH oder N
bedeutet, sowie deren für Pflanzen verträgliche Säureadditionssalze und Metallkomplexe und diese enthaltende Fungizide.

EP 0 330 016 A2

## Neue Azolylmethyloxirane und diese enthaltende Fungizide

Die vorliegende Erfindung betrifft neue Azolverbindungen, Verfahren zu ihrer Herstellung und diese enthaltende Fungizide, sowie Verfahren zur Bekämpfung von Pilzen.

Es ist bekannt, cis-2-(1,2,4-Triazol-1-ylmethyl)-2-(tert.butyl)-3-(4-chlorphenyl)-oxiran zur Bekämpfung von Pilzen zu verwenden (DE-3 218 130.2). Seine Wirkung ist jedoch nicht befriedigend.

Es wurde nun gefunden, daß Azolylmethyloxirane der allgemeinen Formel I

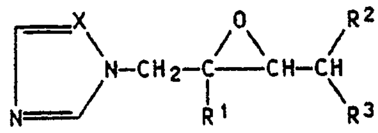

I,

in welcher $R^1$, $R^2$ und $R^3$ $C_1$-$C_{12}$-Alkyl, Benzyl, Naphthyl, Biphenyl, Phenyl, Dioxanyl, Tetrahydropyranyl, Tetrahydrofuranyl, Norbornyl, $C_3$-$C_{12}$-Cycloalkyl oder $C_3$-$C_{12}$-Cycloalkenyl bedeuten, wobei diese Reste gegebenenfalls einfach bis dreifach durch Halogen, Nitro, Phenoxy, Alkyl, Alkoxy, Amino oder Halogenalkyl mit jeweils 1 bis 4 C-Atomen substituiert sind,
X, CH oder N
bedeutet, sowie deren für Pflanzen verträglichen Säureadditionssalze und Metallkomplexe eine bessere fungizide Wirkung, insbesondere gegen Getreidekrankheiten, besitzen als die bekannte Azolverbindung.

Die Verbindungen der Formel I enthalten chirale Zentren und werden im allgemeinen in Form von Diastereomerengemischen erhalten. Die Diastereomeren lassen sich in üblicher Weise, beispielsweise aufgrund ihrer unterschiedlichen Löslichkeit oder durch Säulenchromatographie trennen und in reiner Form isolieren. Aus einem solchen isolierten Diastereomeren kann man mit bekannten Methoden einheitliche Enantiomere erhalten. Als fungizide Mittel kann man sowohl die einheitlichen Diastereomeren bzw. Enantiomere wie auch deren bei der Synthese anfallenden Gemisch verwenden. Alle diese Verbindungen werden von der vorliegenden Erfindung umfaßt.

$R^1$, $R^2$ und $R^3$ bedeuten beispielsweise: Methyl, Ethyl, Isopropyl, n-Propyl, n-Butyl, sec.-Butyl, tert.-Butyl,n-Pentyl, Neopentyl, Hexyl, Octyl, Decyl, Dodecyl, Trifluormethyl, Trichlormethyl, Benzyl, Phenyl, Halogenphenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, Alkoxyphenyl, 2-Methoxyphenyl, 3-Methoxyphenyl, 4-Methoxyphenyl, 2,4-Dimethoxyphenyl, Alkylphenyl, 4-Ethylphenyl, 4-Isopropylphenyl, 4-tert.-Butylphenyl, 4-tert.-Butyloxyphenyl, 2-Chlor-4-fluorphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, 2-Trifluormethylphenyl, 3-Trifluoromethylphenyl, 4-Trifluoromethylphenyl, Cyclopropyl, Cyclobutyl, Cyclopentyl, Cyclohexyl, Cyclooctyl, Cyclododecyl, Tetrahydropyranyl, Tetrahydrofuranyl, 2-Cyclohexenyl, 3-Cyclohexenyl, Norbornyl, 1,3-Dioxan-2-yl, 1,4-Dioxan-2-yl.

Säureadditionssalze sind beispielsweise die Hydrochloride, Bromide, Sulfate, Nitrate, Phosphate, Oxalate oder Dodecylbenzolsulfonate. Die Wirksamkeit der Salze geht auf das Kation zurück, so daß das Anion i.a. beliebig gewählt werden kann. Die erfindungsgemäßen Wirkstoffsalze werden hergestellt durch Umsetzung der Azolylmethyloxirane mit Säuren.

Metallkomplexe der Wirkstoffe I oder ihrer Salze können mit Kupfer, Zink, Zinn, Mangan, Eisen, Kobalt oder Nickel gebildet werden, indem man die Azolylmethyloxirane mit entsprechenden Metallsalzen umsetzt, z.B. mit Kupfersulfat, Zinkchlorid, Zinnchlorid, Mangansulfat, Eisenchlorid, Kobaltsulfat, Nickelsulfat.

Die Verbindungen der Formel I können z.B. hergestellt werden, indem man

a) eine Verbindung der Formel II

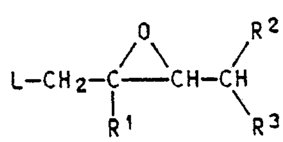

II,

in welcher $R^1$, $R^2$ und $R^3$ die oben angegebenen Bedeutungen haben und L eine nucleophil substituierbare Abgangsgruppe (OH, Halogen) darstellt, mit einer Verbindung der Formel III

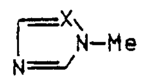

III,

in der Me ein Wasserstoffatom oder ein Metallatom (Na, K) bedeutet und X die oben angegebene Bedeutung hat, zur Umsetzung bringt, oder

b) eine Verbindung der Formel IV

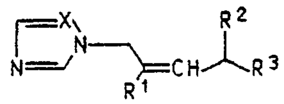

IV,

in welcher $R^1$, $R^2$, $R^3$ und X die oben angegebene Bedeutung haben, in das Epoxid überführt.

Die Reaktion a) erfolgt - falls Me ein Wasserstoffatom bedeutet - gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120 °C. Zu den bevorzugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon, ferner Dimethylsulfoxid, Sulfolan oder entspechende Gemische.

Geeignete Basen, die gegebenenfalls auch als säurebindende Mittel bei der Reaktion verwendet werden können, sind beispielsweise Alkalihydroxid wie Lithium-, Natrium- oder Kaliumhydroxid, Alkalicarbonate wie Natrium, Kalium- oder Caesiumhydrogencarbonat, Pyridin oder 4-Dimethylaminopyridin. Es können aber auch andere übliche Basen verwendet werden.

Als Reaktionsbeschleuniger kommen vorzugsweise Metallhalogenide wie Natriumiodid, oder Kaliumjodid, quartäre Ammoniumsalze wie Tetrabutylammoniumchlorid, -bromid, -jodid oder -hydrogensulfat, Benzyltriethylammoniumchlorid oder -bromid oder Kronenether wie 12-Krone-4, 15-Krone-5, 18-Krone-6, Dibenzo-18-Krone-6 oder Dicyclohexano-18-Krone-6 in Frage.

Die Umsetzung wird im allgemeinen bei Temperaturen zwischen 20 und 150 °C, drucklos oder unter Druck, kontinuierlich oder diskontinuierlich durchgeführt.

Ist Me ein Metallatom wird die Reaktion a) gegebenenfalls in Gegenwart eines Lösungs- oder Verdünnungsmittels und gegebenenfalls unter Zusatz einer starken anorganischen oder organischen Base bei Temperaturen zwischen -10 und 120 °C durchgeführt. Zu den bevorzugten Lösungs- und Ver dünnungsmitteln gehören Amide wie Dimethylformamid, Diethylformamid, Dimethylacetamid, Diethylacetamid, N-Methylpyrrolidon, Hexamethyl-phosphorsäuretriamid, Sulfoxide wie Dimethylsulfoxid und schließlich Sulfolan.

Geeignete Basen, die gegebenenfalls auch als säurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Natrium- oder Kalium-tert.-butoxid, Lithium-, Natrium- oder Kalium-triphenylmethyl und Naphthalinlithium, -natrium oder -kalium.

Für die Reaktion b) kommen als Verdünnungsmittel polare organische Lösungsmittel wie Nitrile, z.B. Acetonitril, Sulfoxide, z.B. Dimethylsulfoxid, Formamide, z.B. Dimethylformamid, Ketone, z.B. Aceton, Ether, z.B. Diethylether, Tetrahydrofuran und insbesondere Chlorkohlenwasserstoffe, z.B. Methylenchlorid und Chloroform, in Frage.

Man arbeitet im allgemeinen zwischen 0 und 100 °C, vorzugsweise bei 20 bis 80 °C. Bei Anwesenheit eines Lösungsmittels wird zweckmäßigerweise bei Siedepunkt des jeweiligen Lösungsmittels gearbeitet.

Die neuen Ausgangsverbindungen II erhält man durch Epoxidierung der entsprechenden Olefine V:

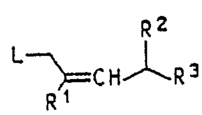

V,

(vgl. G. Dittus in Houben-Weyl-Müller, Methoden der Organischen Chemie, Georg Thieme Verlag, Stuttgart, 1965, Bd. VI, 3, Seite 385)

Die Verbindung V stellt man her, indem man Olefine der Formel VI

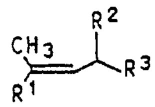 VI

nach bekannten Methoden in Allylposition halogeniert oder oxidiert.

Geeignete Halogenierungsreagenzien sind N-Chlor- und N-Bromsuccinimid in halogenierten Kohlenwasserstoffen wie Tetrachlorkohlenstoff, Trichlorethan oder Methylenchlorid bei Temperaturen zwischen 20 und 100° C. Zur Allyloxidation verwendet man Perester wie Perbenzoesäure-tert.-butylester oder Peressigsäure-tert.-butylester in Anwesenheit eines Schwermetallsalzes wie z.B. Kupfer-I-chlorid oder Kupfer-I-bromid. Man arbeitet in inerten Lösungsmitteln bei Temperaturen zwischen 10 und 100° C.

Die so erhaltenen Allylhalogenide bzw. -alkohole V werden anschließend in die entsprechenden Epoxide II (L = Halogen, OH) übergeführt. Dazu oxidiert man die Olefine V mit Peroxycarbonsäuren wie Perbenzoesäure, 3-Chlorperbenzoesäure, 4-Nitroperbenzoesäure, Monoperphthalsäure, Peressigsäure, Perpropionsäure, Permaleinsäure, Monoperbernsteinsäure, Perpelargonsäure oder Trifluorperessigsäure in indifferenten Lösungsmitteln, vorzugsweise chlorierten Kohlenwasserstoffen, z.B. Methylenchlorid, Chloroform, Tetrachlorkohlenstoff, Dichlorethan, oder gegebenenfalls auch in Essigsäure, Essigester, Aceton oder Dimethylformamid, gegebenenfalls in Gegenwart eines Puffers wie Natriumacetat, Natriumcarbonat, Dinatriumhydrogenphosphat. Man arbeitet zwischen 10 und 100 °C und katalysiert die Reaktion gegebenenfalls z.B. mit Jod, Natriumwolframat oder Licht. Zur Oxidation eignen sich auch alkalische Lösungen von Wasserstoffperoxid (ca. 30 %ig) in Methanol, Ethanol, Aceton oder Acetonitril bei 25 bis 30 °C sowie Alkylhydroperoxide, z.B. tert.-Butylhydroperoxid, unter Zusatz eines Katalysators, z.B Natriumwolframat, Perwolframsäure, Molybdänhexacarbonyl oder Vanadylacetylacetonat. Die genannten Oxidationsmittel lassen sich z.T. in situ erzeugen.

Während die so erhaltenen Epoxihalogenide (L = Halogen) gemäß Verfahren a) direkt umgesetzt werden können, überführt man die entsprechenden Epoxialkohole II (L = OH) in reaktive Ester, die mit den Verbindungen III gemäß Verfahren a) umgesetzt werden.

Die Darstellung der reaktiven Ester, die mit III umgesetzt werden, erfolgt nach allgemein bekannten Methoden (Houben-Weyl-Müller, Methoden der organischen Chemie, Georg Thieme Verlag, Stuttgart, 1955, Band 9, Seiten 388, 663, 671). Solche Ester sind beispielsweise Methansulfonsäureester, Trifluormethansulfonsäureester, 2,2,2-Trifluorethansulfonsäureester, Nonafluorbutansulfonsäureester, 4-Methylbenzosulfonsäureester, 4-Brombenzolsulfonsäureester, 4-Nitrobenzolsulfonsäureester oder Benzolsulfonsäureester.

Die Verbindungen V lassen sich entsprechend allgemein bekannten Verfahren zur Olefinsynthese (Houben-Weyl-Müller, Methoden der organischen Chemie, Georg Thieme Verlag, Stuttgart, 1972 Bd. V, 1b) herstellen.

Die folgenden Beispiele erläutern die Herstellung der Wirkstoffe.

I. Herstellung der Ausgangsstoffe

Vorschrift A

Zu einer Lösung von 41,5 g 2-(4-Fluorphenyl)-butanal in 200 ml Methanol werden 4,2 g Natriumhydroxid in 30 ml Wasser gegeben. Das Reaktionsgemisch wird auf 10 °C gekühlt und schnell 36 g 4-Fluorphenylacetaldehyd zugetropft, wobei die Temperatur in der Lösung auf 30 °C ansteigt. Nach zweistündigem Rühren bei Raumtemperatur (20 °C) wird der farblosen Reaktionslösung 200 ml Wasser zugesetzt und die entstandene Emulsion mit Methyl-tert.-butylether ausgeschüttelt. Die organische Phase wird abgetrennt, über Natriumsulfat getrocknet und im Vakuum eingedampft. Bei der anschließenden Destillation des verbleibenden Rückstandes werden bei 1 mbar und 103° C 34,3 g (48 %) E/Z-4-R,S-2-(4-Fluorphenyl)-4-(4-fluorphenyl)-hex-2-enal erhalten.

Vorschrift B

34,3 g E/Z-4-R,S-2-(4-Fluorphenyl)-4-(4-fluorphenyl)-hex-2-enal werden in 200 ml Methanol gelöst und 0,85 ml Natronlauge (konz.) zugesetzt. Die Reaktionslösung wird bei 0° C gerührt, während 10,2 g Wasserstoffperoxid (ca. 50 gew.%ig) langsam zugetropft werden, wobei die Innentemperatur von 30 °C nicht überschritten wird. Nach beendeter Zugabe wird sechs Stunden bei Raumtemperatur gerührt und anschließend 1,85 g Natriumborhydrid zugegeben, das in wenig 10 %iger Natronlauge gelöst war. Nachdem das Reaktionsgemisch weitere 18 Stunden bei Raumtemperatur gerührt hat, wird der Lösung 100 ml Wasser zugesetzt und die entstandene Emulsion mit Methylenchlorid ausgeschüttelt. Die isolierte organische Phase wird über Natriumsulfat getrocknet und eingeengt. Man erhält 31 g (85 %) cis/trans-1′-R,S-2-Hydroxymethyl-2-(4-fluorphenyl)-3-[1-(4-fluorphenyl)-prop-1-yl]-oxiran.

Vorschrift C

Zu einer Lösung von 31 g cis/trans-1′R,S-2-Hydroxymethyl-2-(4-fluorphenyl)-3-[1-(4-fluorphenyl)-prop-1-yl]-oxiran in 150 ml Methylenchlorid und 20 g Triethylamin werden bei Raumtemperatur 21 g 4-Methylbenzolsulfonsäurechlorid zugesetzt. Nach 24 Stunden wird das Reaktionsgemisch mit wäßriger Natriumhydrogencarbonat-Lösung und Wasser gewaschen, über Natriumsulfat getrocknet und im Vakuum eingedampft. Aus dem Rückstand erhielt man 43,5 g (95 %) cis/trans-1′R,S-2-(4-Methylphenylsulfonyloxymethyl)-2-(4-fluor phenyl)-3-[1-(4-fluorphenyl)-prop-1-yl]-oxiran, das anschließend mit Triazol gemäß dem folgenden Beispiel weiter verarbeitet wurde.

II. Herstellung der Endprodukte

Beispiel 1

Eine Lösung von 14 g 1,2,4-Triazol in 150 ml N-Methyl-pyrrolidon wird mit 8 g Natriumhydroxid versetzt und für 30 Minuten auf 50° C erwärmt. Nachdem das Reaktionsgemisch auf Raumtemperatur gekühlt wurde, wird der Lösung 43,5 g cis/trans-1′-R,S-2-(4-Methylphenylsulfonyloxymethyl)-2-(4-fluorphenyl)-3-[1-(4-fluorphenyl)-prop-1-yl]-oxiran, das in 100 ml N-Methyl-pyrrolidon gelöst ist, langsam zugetropft und 12 Stunden bei Raumtemperatur gerührt. Anschließend wird 200 ml Wasser zugegeben und mehrmals mit Methyl-tert.-butylether extrahiert; die organische Phase mit Wasser gewaschen, über Natriumsulfat getrocknet und im Vakuum eingeengt. Man erhält aus Methyl-tert.-butylether/n-Hexan 36,3 g (78 %) cis/trans-1′R,S-2-(1,2,4-Triazol-1-yl-methyl)-2-(4-fluorphenyl)-3-[1-(4-fluorphenyl)-prop-1-yl]oxiran mit dem Schmelzpunkt 123 bis 127° C (Verbindung Nr. 1).

Entsprechend Beispiel 1 können die in der Tabelle aufgeführten Verbindungen hergestellt werden.

Tabelle

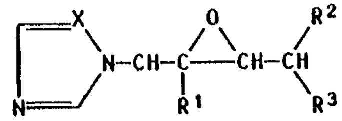

| Bsp. | $R^1$ | $R^2$ | $R^3$ | X | Schmp./IR |
|---|---|---|---|---|---|
| 1 | 4-F-$C_6H_4$ | $C_2H_5$ | 4-F-$C_6H_4$ | N | 123-127°C |
| 2 | 4-F-$C_6H_4$ | $CH_3$ | 4-F-$C_6H_4$ | N | - |
| 3 | 4-F-$C_6H_4$ | $C_3H_7$ | 4-F-$C_6H_4$ | N | - |
| 4 | 4-F-$C_6H_4$ | $C_4H_9$ | 4-F-$C_6H_4$ | N | IR: 2958, 1605, 1509, 1225, 837 cm $^{-cm}$ |
| 5 | 4-F-$C_6H_4$ | tert.-butyl | 4-F-$C_6H_4$ | N | - |
| 6 | 4-F-$C_6H_4$ | benzyl | 4-F-$C_6H_4$ | N | - |
| 7 | 4-F-$C_6H_4$ | 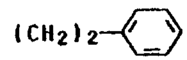 | 4-F-$C_6H_4$ | N | - |
| 8 | 4-F-$C_6H_4$ | 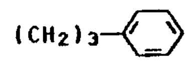 | 4-F-$C_6H_4$ | N | - |
| 9 | 4-F-$C_6H_4$ | 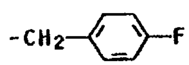 | 4-F-$C_6H_4$ | N | - |
| 10 | 4-F-$C_6H_4$ | 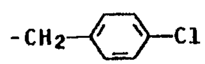 | 4-F-$C_6H_4$ | N | - |
| 11 | 4-F-$C_6H_4$ | 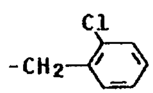 | 4-F-$C_6H_4$ | N | - |
| 12 | 4-F-$C_6H_4$ | 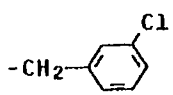 | 4-F-$C_6H_4$ | N | - |
| 13 | 4-F-$C_6H_4$ | 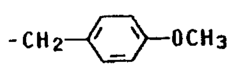 | 4-F-$C_6H_4$ | N | - |

Tabelle (Fortsetzung)

| Bsp. | $R^1$ | $R^2$ | $R^3$ | X | Schmp./IR |
|---|---|---|---|---|---|
| 14 | 4-F-$C_6H_4$ | 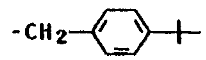 | 4-F-$C_6H_4$ | N | - |
| 15 | 4-F-$C_6H_4$ | 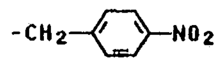 | 4-F-$C_6H_4$ | N | - |
| 16 | 4-F-$C_6H_4$ | 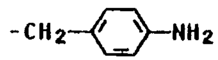 | 4-F-$C_6H_4$ | N | - |
| 17 | 4-F-$C_6H_4$ | 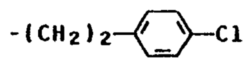 | 4-F-$C_6H_4$ | N | - |
| 18 | 4-F-$C_6H_4$ | 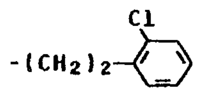 | 4-F-$C_6H_4$ | N | - |
| 19 | 4-F-$C_6H_4$ | 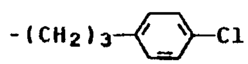 | 4-F-$C_6H_4$ | N | - |
| 20 | 4-F-$C_6H_4$ | 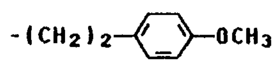 | 4-F-$C_6H_4$ | N | - |
| 21 | 4-F-$C_6H_4$ | $CH_3$ | 2-F-$C_6H_4$ | N | - |
| 22 | 4-F-$C_6H_4$ | $C_2H_5$ | 2-F-$C_6H_4$ | N | - |
| 23 | 4-F-$C_6H_4$ | $C_3H_7$ | 2-F-$C_6H_4$ | N | - |
| 24 | 4-F-$C_6H_4$ | $C_4H_9$ | 2-F-$C_6H_4$ | N | - |
| 25 | 4-F-$C_6H_4$ | iso-$C_3H_7$ | 2-F-$C_6H_4$ | N | - |
| 26 | 4-F-$C_6H_4$ | tert.-butyl | 2-F-$C_6H_4$ | N | - |
| 27 | 4-F-$C_6H_4$ | benzyl | 2-F-$C_6H_4$ | N | - |
| 28 | 4-F-$C_6H_4$ | 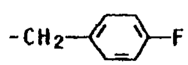 | 2-F-$C_6H_4$ | N | - |

Tabelle (Fortsetzung)

| Bsp. | $R^1$ | $R^2$ | $R^3$ | X | Schmp./IR |
|---|---|---|---|---|---|
| 29 | $4-F-C_6H_4$ | 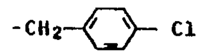 | $2-F-C_6H_4$ | N | - |
| 30 | $4-F-C_6H_4$ | 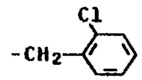 | $2-F-C_6H_4$ | N | - |
| 31 | $4-F-C_6H_4$ | 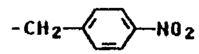 | $2-F-C_6H_4$ | N | - |
| 32 | $4-F-C_6H_4$ | 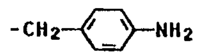 | $2-F-C_6H_4$ | N | - |
| 33 | $4-F-C_6H_4$ | $CH_3$ | $2-Cl-C_6H_4$ | N | - |
| 34 | $4-F-C_6H_4$ | $C_2H_5$ | $2-Cl-C_6H_4$ | N | - |
| 35 | $4-F-C_6H_4$ | $C_3H_7$ | $2-Cl-C_6H_4$ | N | - |
| 36 | $4-F-C_6H_4$ | $C_4H_9$ | $2-Cl-C_6H_4$ | N | - |
| 37 | $4-F-C_6H_4$ | iso-$C_3H_7$ | $2-Cl-C_6H_4$ | N | - |
| 38 | $4-F-C_6H_4$ | tert.-butyl | $2-Cl-C_6H_4$ | N | - |
| 39 | $4-F-C_6H_4$ | benzyl | $2-Cl-C_6H_4$ | N | - |
| 40 | $4-F-C_6H_4$ | 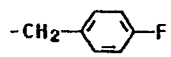 | $2-Cl-C_6H_4$ | N | - |
| 41 | $4-F-C_6H_4$ | 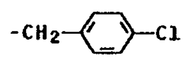 | $2-Cl-C_6H_4$ | N | - |
| 42 | $4-F-C_6H_4$ | 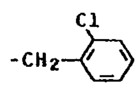 | $2-Cl-C_6H_4$ | N | - |
| 43 | $4-F-C_6H_4$ | 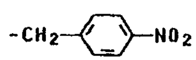 | $2-Cl-C_6H_4$ | N | - |
| 44 | $4-F-C_6H_4$ | 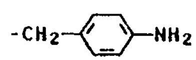 | $2-Cl-C_6H_4$ | N | - |

Tabelle (Fortsetzung)

| Bsp. | $R^1$ | $R^2$ | $R^3$ | X | Schmp./IR |
|---|---|---|---|---|---|
| 45 | $4-F-C_6H_4$ | $CH_3$ | $3-Cl-C_6H_4$ | N | m |
| 46 | $4-F-C_6H_4$ | $C_2H_5$ | $3-Cl-C_6H_4$ | N | - |
| 47 | $4-F-C_6H_4$ | $C_3H_7$ | $3-Cl-C_6H_4$ | N | - |
| 48 | $4-F-C_6H_4$ | iso-$C_3H_7$ | $3-Cl-C_6H_4$ | N | - |
| 49 | $4-F-C_6H_4$ | tert.-butyl | $3-Cl-C_6H_4$ | N | - |
| 50 | $4-F-C_6H_4$ | benzyl | $3-Cl-C_6H_4$ | N | - |
| 51 | $4-F-C_6H_4$ | 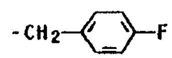 | $3-Cl-C_6H_4$ | N | - |
| 52 | $4-F-C_6H_4$ | 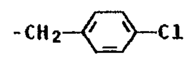 | $3-Cl-C_6H_4$ | N | - |
| 53 | $4-F-C_6H_4$ | $CH_3$ | $4-Cl-C_6H_4$ | N | - |
| 54 | $4-F-C_6H_4$ | $C_2H_5$ | $4-Cl-C_6H_4$ | N | - |
| 55 | $4-F-C_6H_4$ | iso-$C_3H_7$ | $4-Cl-C_6H_4$ | N | - |
| 56 | $4-F-C_6H_4$ | tert.-butyl | $4-Cl-C_6H_4$ | N | - |
| 57 | $4-F-C_6H_4$ | benzyl | $4-Cl-C_6H_4$ | N | - |
| 58 | $4-F-C_6H_4$ | 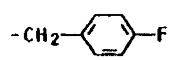 | $4-Cl-C_6H_4$ | N | - |
| 59 | $4-F-C_6H_4$ | 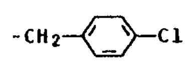 | $4-Cl-C_6H_4$ | N | - |
| 60 | $4-F-C_6H_4$ | 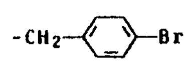 | $4-Cl-C_6H_4$ | N | - |
| 61 | $4-F-C_6H_4$ | $CH_3$ | $2,4-Cl_2-C_6H_3$ | N | - |
| 62 | $4-F-C_6H_4$ | $C_2H_5$ | $2,4-Cl_2-C_6H_3$ | N | - |
| 63 | $4-F-C_6H_4$ | iso-$C_3H_7$ | $2,4-Cl_2-C_6H_3$ | N | - |
| 64 | $4-F-C_6H_4$ | tert.-butyl | $2,4-Cl_2-C_6H_3$ | N | - |
| 65 | $4-F-C_6H_4$ | benzyl | $2,4-Cl_2-C_6H_3$ | N | - |

Tabelle (Fortsetzung)

| Bsp. | $R^1$ | $R^2$ | $R^3$ | X | Schmp./IR |
|---|---|---|---|---|---|
| 66 | 4-F-$C_6H_4$ | 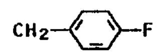 | 2,4-$Cl_2$-$C_6H_3$ | N | - |
| 67 | 4-F-$C_6H_4$ | 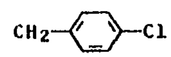 | 2,4-$Cl_2$-$C_6H_3$ | N | - |
| 68 | 4-F-$C_6H_4$ | $C_2H_5$ | 4-Br-$C_6H_4$ | N | - |
| 69 | 4-F-$C_6H_4$ | $CH_3$ | 2-$OCH_3$-$C_6H_4$ | N | - |
| 70 | 4-F-$C_6H_4$ | $C_3H_7$ | 4-$NO_2$-$C_6H_4$ | N | - |
| 71 | 4-F-$C_6H_4$ | benzyl | 2-$CF_3$-$C_6H_4$ | N | - |
| 72 | 4-F-$C_6H_4$ | 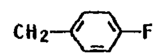 | 2-Cl-4-F-$C_6H_3$ | N | - |
| 73 | 4-F-$C_6H_4$ | 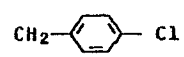 | 2-Cl-6-F-$C_6H_3$ | N | - |
| 74 | 4-Cl-$C_6H_4$ | $CH_3$ | 4-F-$C_6H_4$ | N | - |
| 75 | 4-Cl-$C_6H_4$ | $C_2H_5$ | 4-F-$C_6H_4$ | N | - |
| 76 | 4-Cl-$C_6H_4$ | benzyl | 4-F-$C_6H_4$ | N | - |
| 77 | 4-Cl-$C_6H_4$ | $CH_3$ | 2-F-$C_6H_4$ | N | - |
| 78 | 4-Cl-$C_6H_4$ | $C_2H_5$ | 2-F-$C_6H_4$ | N | - |
| 79 | 4-Cl-$C_6H_4$ | benzyl | 2-F-$C_6H_4$ | N | - |
| 80 | 4-Cl-$C_6H_4$ | $CH_3$ | 2-Cl-$C_6H_4$ | N | - |
| 81 | 4-Cl-$C_6H_4$ | 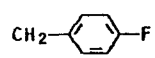 | 2-Cl-$C_6H_4$ | N | - |
| 82 | 4-Cl-$C_6H_4$ | $C_2H_5$ | 3-Cl-$C_6H_4$ | N | - |
| 83 | 4-Cl-$C_6H_4$ | 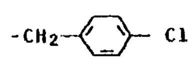 | 3-Cl-$C_6H_4$ | N | - |
| 84 | 4-Cl-$C_6H_4$ | $C_2H_5$ | 4-Cl-$C_6H_4$ | N | - |
| 85 | 4-Cl-$C_6H_4$ | 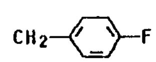 | 4-Cl-$C_6H_4$ | N | - |

Tabelle (Fortsetzung)

| Bsp. | $R^1$ | $R^2$ | $R^3$ | X | Schmp./IR |
|---|---|---|---|---|---|
| 86 | 4-Cl-$C_6H_4$ | $CH_3$ | 2,4-$Cl_2$-$C_6H_3$ | N | - |
| 87 | 4-Cl-$C_6H_4$ | benzyl | 2,4-$Cl_2$-$C_6H_3$ | N | - |
| 88 | 2-Cl-$C_6H_4$ | $CH_3$ | 4-F-$C_6H_4$ | N | - |
| 89 | 2-Cl-$C_6H_4$ | benzyl | 4-F-$C_6H_4$ | N | - |
| 90 | 2-Cl-$C_6H_4$ | $CH_3$ | 2-F-$C_6H_4$ | N | - |
| 91 | 2-Cl-$C_6H_4$ | 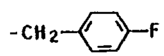 | 2-F-$C_6H_4$ | N | - |
| 92 | 2-Cl-$C_6H_4$ | $C_2H_5$ | 2-Cl-$C_6H_4$ | N | - |
| 93 | 2-Cl-$C_6H_4$ | benzyl | 2-Cl-$C_6H_4$ | N | - |
| 94 | 2-Cl-$C_6H_4$ | $CH_3$ | 4-Cl-$C_6H_4$ | N | - |
| 95 | 2-Cl-$C_6H_4$ | 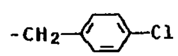 | 4-Cl-$C_6H_4$ | N | - |
| 96 | 2-Cl-$C_6H_4$ | $CH_3$ | 2,4-$Cl_2$-$C_6H_3$ | N | - |
| 97 | 2-Cl-$C_6H_4$ | benzyl | 2,4-$Cl_2$-$C_6H_3$ | N | - |
| 98 | 2-Cl-$C_6H_4$ | $CH_3$ | 3-Cl-$C_6H_4$ | N | - |
| 99 | 2-Cl-$C_6H_4$ | benzyl | 3-Cl-$C_6H_4$ | N | - |
| 100 | 2,4-$Cl_2$-$C_6H_3$ | $C_2H_5$ | 4-F-$C_6H_4$ | N | - |
| 101 | 2,4-$Cl_2$-$C_6H_3$ | benzyl | 4-F-$C_6H_4$ | N | - |
| 102 | 2,4-$Cl_2$-$C_6H_3$ | $C_2H_5$ | 2-Cl-$C_6H_4$ | N | - |
| 103 | 2,4-$Cl_2$-$C_6H_3$ | benzyl | 2-Cl-$C_6H_4$ | N | - |
| 104 | 2,4-$Cl_2$-$C_6H_3$ | $CH_3$ | 4-Cl-$C_6H_4$ | N | - |
| 105 | 2,4-$Cl_2$-$C_6H_3$ | benzyl | 4-Cl-$C_6H_4$ | N | - |
| 106 | Cyclohexyl | $C_2H_5$ | 4-F-$C_6H_4$ | N | - |
| 107 | Cyclohexyl | benzyl | 4-F-$C_6H_4$ | N | - |
| 108 | Cyclohexyl | $C_2H_5$ | 2-Cl-$C_6H_4$ | N | - |
| 109 | Cyclohexyl | benzyl | 2-Cl-$C_6H_4$ | N | - |
| 110 | Cyclohexyl | $CH_3$ | 4-Cl-$C_6H_4$ | N | - |

Tabelle (Fortsetzung)

| Bsp. | $R^1$ | $R^2$ | $R^3$ | X | Schmp./IR |
|---|---|---|---|---|---|
| 111 | Cyclohexyl | benzyl | 4-Cl-$C_6H_4$ | N | - |
| 112 | 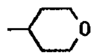 | $CH_3$ | 4-F-$C_6H_4$ | N | - |
| 113 | 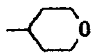 | benzyl | 4-F-$C_6H_4$ | N | - |
| 114 | 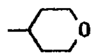 | $CH_3$ | 2-Cl-$C_6H_4$ | N | - |
| 115 | 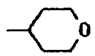 | benzyl | 2-Cl-$C_6H_4$ | N | - |
| 116 | 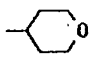 | $CH_3$ | 4-Cl-$C_6H_4$ | N | - |
| 117 | 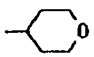 | benzyl | 4-Cl-$C_6H_4$ | N | - |
| 118 | tert.-butyl | $CH_3$ | 4-F-$C_6H_4$ | N | - |
| 119 | tert.-butyl | benzyl | 4-F-$C_6H_4$ | N | - |
| 120 | tert.-butyl | $CH_3$ | 2-Cl-$C_6H_4$ | N | - |
| 121 | tert.-butyl | benzyl | 2-Cl-$C_6H_4$ | N | - |
| 122 | tert.-butyl | $CH_3$ | 4-Cl-$C_6H_4$ | N | - |
| 123 | tert.-butyl | benzyl | 4-Cl-$C_6H_4$ | N | - |
| 124 | $CH_3$ | $CH_3$ | 4-F-$C_6H_4$ | N | - |
| 125 | $CH_3$ | benzyl | 4-F-$C_6H_4$ | N | - |
| 126 | 4-F-$C_6H_4$ | $C_2H_5$ | 4-F-$C_6H_4$ | CH | - |
| 127 | 4-F-$C_6H_4$ | $CH_3$ | 4-F-$C_6H_4$ | CH | - |
| 128 | 4-F-$C_6H_4$ | $C_3H_7$ | 4-F-$C_6H_4$ | CH | - |
| 129 | 4-F-$C_6H_4$ | $C_4H_9$ | 4-F-$C_6H_4$ | CH | - |

Tabelle (Fortsetzung)

| Bsp. | $R^1$ | $R^2$ | $R^3$ | X | Schmp./IR |
|---|---|---|---|---|---|
| 130 | 4-F-$C_6H_4$ | 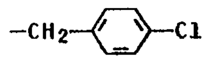 | 4-F-$C_6H_4$ | CH | - |
| 131 | 4-F-$C_6H_4$ | $C_2H_5$ | 2-F-$C_6H_4$ | CH | - |
| 132 | 4-F-$C_6H_4$ | iso-$C_3H_7$ | 4-F-$C_6H_4$ | CH | - |
| 133 | 4-F-$C_6H_4$ | 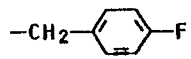 | 4-F-$C_6H_4$ | CH | - |
| 134 | 4-F-$C_6H_4$ | $C_2H_5$ | 2-Cl-$C_6H_4$ | CH | - |
| 135 | 4-F-$C_6H_4$ | 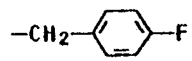 | 2-Cl-$C_6H_4$ | CH | - |
| 136 | 4-F-$C_6H_4$ | $CH_3$ | 3-Cl-$C_6H_4$ | CH | - |
| 137 | 4-F-$C_6H_4$ | 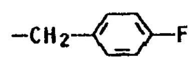 | 3-Cl-$C_6H_4$ | CH | - |
| 138 | 4-F-$C_6H_4$ | $C_2H_5$ | 4-Cl-$C_6H_4$ | CH | - |
| 139 | 4-F-$C_6H_4$ | 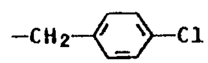 | 4-Cl-$C_6H_4$ | CH | - |
| 140 | 4-F-$C_6H_4$ | $C_2H_5$ | 2,4-$Cl_2$-$C_6H_3$ | CH | - |
| 141 | 4-F-$C_6H_4$ | $C_2H_5$ | 4-Br-$C_6H_4$ | CH | - |
| 142 | 4-Cl-$C_6H_4$ | $C_2H_5$ | 4-F-$C_6H_4$ | CH | - |
| 143 | 4-Cl-$C_6H_4$ | $C_2H_5$ | 3-Cl-$C_6H_4$ | CH | - |
| 144 | 4-Cl-$C_6H_4$ | $CH_3$ | 2,4-$Cl_2$-$C_6H_3$ | CH | - |
| 145 | 2-Cl-$C_6H_4$ | $C_2H_5$ | 2-Cl-$C_6H_4$ | CH | - |
| 146 | Cyclohexyl | benzyl | 4-F-$C_6H_4$ | CH | - |
| 147 | Cyclohexyl | $C_2H_5$ | 2-Cl-$C_6H_4$ | CH | - |
| 148 | 4-F-$C_6H_4$ | $C_7H_{15}$ | 2-Cl-$C_6H_4$ | N | 2954, 2928, 1507, 1273, 1140, 755 $cm^{-1}$ |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide, Erysiphe cichoracearum und Sphaerotheca fuliginea an Kürbisgewächsen, Podosphaera leucotricha an Äpfeln
Uncinula necator an Reben,
Puccinia-Arten an Getreide
Rhizoctonia-Arten an Baumwolle und Rasen,
Ustilago-Arten an Getreide und Zuckerrohr
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara Viticola an Reben
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Ver teilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 1 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungs produktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 1 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 1 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 1 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 1 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 1 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 34 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:
Schwefel,
Dithiocarbamate und deren Derivate, wie
Ferridimethyldithiocarbamat,
Zinkdimethyldithiocarbamat,
Zinkethylenbisdithiocarbamat
Manganethylenbisdithiocarbamat
Mangan-Zink-ethylendiamin-bis-dithiocarbamat
Tetramethylthiuramdisulfide,
Ammoniak-Komplex von Zink-(N,N-ethylen-bis-dithiocarbamat)
Ammoniak-
Komplex von Zink-(N,N′-propylen-bis-dithiocarbamat)
Zink-(N,N′-propylen-bis-dithiocarbamat),
N,N′-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester

heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N′,N′-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N′-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat
DL-N-(2,6-Dimethyl-phenyl)-N-(2′-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,

3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

## Anwendungsbeispiele

Als Vergleichswirkstoff wurde cis-2-(1,2,4-Triazol-1-ylmethyl)-2-(tert.-butyl)-3-(4-chlorphenyl)-oxiran (A) - bekannt aus DE-3 218 130.2 benutzt.

### Anwendungsbeispiel 1

Wirksamkeit gegen Weizenbraunrost

Blätter von in Töpfen gewachsenen Weizensämlingen der Sorte "Frühgold" wurden mit Sporen des Braunrostes (Puccinia recondita) bestäubt. Danach wurden die Töpfe für 24 Stunden bei 20 bis 22 °C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Abtrocknen des Spritzbelages wurden die Versuchspflanzen im Gewächshaus bei Temperaturen zwischen 20 und 22 °C und 65 bis 70 % relativer Luftfeuchte aufgestellt. Nach 8 Tagen wurde das Ausmaß der Rostpilzentwicklung auf den Blättern ermittelt.

Das Ergebnis zeigt, daß der Wirkstoff 1 bei der Anwendung als 0,025 %ige ( Gew.%) Spritzbrühe eine bessere fungizide Wirkung zeigt (97 %) als der bekannte Vergleichswirkstoff A (60 %).

### Anwendungsbeispiel 2

Wirksamkeit gegen Botrytis cinerea an Paprika

Paprikasämlinge der Sorte "Neusiedler Ideal Elite" wurden, nachdem sich 4 bis 5 Blätter gut entwickelt hatten, mit wäßrigen Suspensionen, die 80 % Wirkstoff und 20 % Emulgiermittel in der Trockensubstanz enthielten, tropfnaß gespritz. Nach dem Antrocknen des Spritzbelages wurden die Pflanzen mit einer Konidienaufschwemmung des Pilzes Botrytis cinerea besprüht und bei 22 bis 24 °C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Das Ergebnis zeigt, daß der Wirkstoff 1 bei der Anwendung als 0,05 %ige Spritzbrühe eine bessere fungizide Wirkung zeigt (90 %) als der bekannte Vergleichswirkstoff A (60 %).

### Anwendungsbeispiel 3

Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18 °C gestellt. Anschließend wurden die Pflanzen im Gewächshaus bei 20 - 22 °C und 70 % relativer Luftfeuchtigkeit für weitere 5 Tage kultiviert.

Dann wurde das Ausmaß der Symptomentwicklung ermittelt.

Das Ergebnis zeigt, daß der Wirkstoff 1 bei der Anwendung als 0,05 %ige Spritzbrühe eine sehr gute fungizide Wirkung (97 %) zeigt.

## Ansprüche

1. Azolylmethyloxirane der allgemeinen Formel I

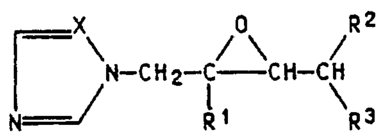

I,

in welcher $R^1$, $R^2$ und $R^3$ $C_1$-$C_{12}$-Alkyl, Benzyl, Naphthyl, Biphenyl, Dioxanyl, Phenyl, Tetrahydropyranyl, Tetrahydrofuranyl, Norbornyl, $C_3$-$C_{12}$-Cycloalkyl oder $C_3$-$C_{12}$-Cycloalkenyl bedeuten, wobei diese Reste gegebenenfalls durch Halogen, Nitro, Phenoxy, Alkyl, Alkoxy, Amino oder Halogenalkyl mit jeweils 1 bis 4 C-Atomen substituiert sind,
X, CH oder N
bedeutet, sowie deren für Pflanzen verträgliche Säureadditionssalze und Metallkomplexe.

2. Azolylmethyloxiran gemäß Anspruch 1, dadurch gekennzeichnet, daß $R^1$ Phenyl, $C_1$-$C_4$-Alkyl, Tetrahydropyranyl, $R^2$ Benzyl, $C_1$-$C_4$-Alkyl, $R^3$ Phenyl bedeuten, wobei diese Reste gegebenenfalls durch Halogen, Nitro, Phenoxy, Alkyl, Alkoxy, Amino oder Halogenalkyl mit jeweils 1 bis 4 C-Atomen substituiert sind.

3. Verfahren zur Herstellung der Azolylmethyloxirane der Formel I gemäß Anspruch 1, dadurch gekennzeichnet, daß man

a) eine Verbindung der Formel II

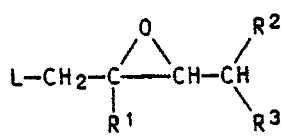

II,

in welcher $R^1$, $R^2$ und $R^3$ die oben angegebenen Bedeutungen haben und L eine nucleophil substituierbare Abgangsgruppe darstellt, mit einer Verbindung der Formel III

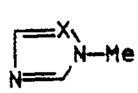

III,

in der Me ein Wasserstoffatom oder ein Metallatom bedeutet und X die oben angegebene Bedeutung hat, zur Umsetzung bringt, oder

b) eine Verbindung der Formel IV

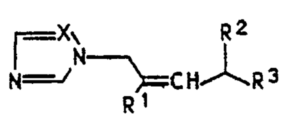

IV,

in welcher $R^1$, $R^2$, $R^3$ und X die oben angegebene Bedeutung haben, in das Epoxid überführt.

4. Fungizides Mittel, enthaltend einen Trägerstoff und ein Azolylmethyloxiran der allgemeinen Formel I

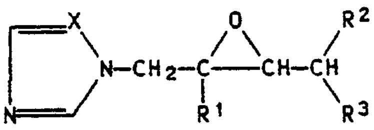

I,

in welcher $R^1$, $R^2$ und $R^3$ $C_1$-$C_{12}$-Alkyl, Benzyl, Naphthyl, Biphenyl, Dioxanyl, Phenyl, Tetrahydropyranyl, Tetrahydrofuranyl, Norbornyl, $C_3$-$C_{12}$-Cycloalkyl oder $C_3$-$C_{12}$-Cycloalkenyl bedeuten, wobei diese Reste gegebenenfalls durch Halogen, Nitro, Phenoxy, Alkyl, Alkoxy, Amino oder Halogenalkyl mit jeweils 1 bis 4 C-Atomen substituiert sind,
X, CH oder N
bedeutet, oder dessen für Pflanzen verträgliches Säureadditionssalz oder Metallkomplex.

5. Verfahren zur Bekämpfung von Pilzen, dadurch gekennzeichnet, daß man eine fungizid wirksame Menge eines Azolylmethyloxirans der allgemeinen Formel I

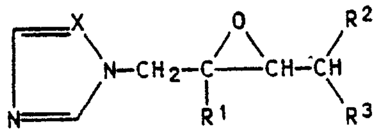

I,

in welcher $R^1$, $R^2$ und $R^3$ $C_1$-$C_{12}$-Alkyl, Benzyl, Naphthyl, Biphenyl, Dioxanyl, Phenyl, Tetrahydropyranyl, Tetrahydrofuranyl, Norbornyl, $C_3$-$C_{12}$-Cycloalkyl oder $C_3$-$C_{12}$-Cycloalkenyl bedeuten, wobei diese Reste gegebenenfalls durch Halogen, Nitro, Phenoxy, Alkyl, Alkoxy, Amino oder Halogenalkyl mit jeweils 1 bis 4 C-Atomen substituiert sind,
X, CH oder N
bedeutet, oder dessen für Pflanzen verträgliches Säureadditionssalz oder Metallkomplex auf die Pilze oder auf durch Pilzbefall bedrohte Materialien, Flächen, Pflanzen oder Saatgüter einwirken läßt.

6. Verbindungen gemäß Anspruch 1, dadurch gekennzeichnet, daß
$R^1$ 4-Fluorphenyl,
$R^2$ Ethyl,
$R^3$ 4-Fluorphenyl und
X den Rest N bedeuten.